# EUROPEAN PATENT APPLICATION

(11) **EP 1 897 542 A1**
(43) Date of publication of application: **12.03.2008**
(21) Application number: 06291410.6
(22) Date of filing: 07.09.2006
(51) Int. Cl.: A61K 31/47, A61P 35/00, C07D 215/22, C07D 241/44, A61K 9/08, A61K 47/40

(54) **Aqueous formulation comprising an antitumor agent**

(71) Applicant: Sanofi-Aventis, 75013 Paris (FR)
(72) Inventor: Jiping, Liu, Malvern, PA 19355 (US); Dorchies, Olivier, 75011 Paris (FR); Rocco, William L., Reading, PA 19606 (US)

(57) **Abstract**

Disclosed are compositions and pharmaceutical formulations comprising (2R)-2-[4-(7-bromo-2-quinolyloxy)phenoxy]propanoic acid, and methods of use thereof.

## Description

The present invention relates to the active agent (2R)-2-[4-(7-bromo-2-quinolyloxy)phenoxy]propanoic acid, and, in particular, a novel pharmaceutical formulation for parenteral administration of (2R)-2-[4-(7-bromo-2-quinolyloxy)phenoxy]propanoic acid or a pharmaceutically acceptable salt thereof.

### BACKGROUND OF THE INVENTION

(2R)-2-[4-(7-bromo-2-quinolyloxy)phenoxy]propanoic acid, which has the structure of Formula (I): 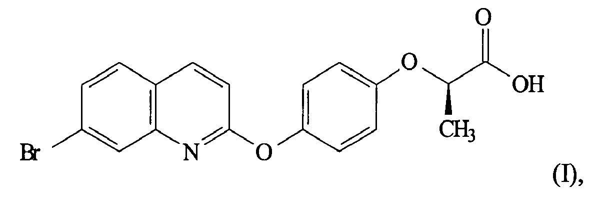 has been shown to be particularly advantageous as an anticancer agent. The preparation, physical properties and beneficial pharmacological properties of (2R)-2-[4-(7-bromo-2-quinolyloxy)phenoxy]propanoic acid are described in, for example, U.S. Patent No. 6,867,219, which is incorporated by reference herein in its entirety.

(2R)-2-[4-(7-Bromo-2-quinolyloxy)phenoxy]propanoic acid may be administered as a liquid parenteral formulation. In preliminary animal toxicological studies, (2R)-2-[4-(7-bromo-2-quinolyloxy)phenoxy]propanoic acid was found to cause a degree of hemolysis when infused at 10 mg/mL or greater as a simple phosphate buffer formulation. Since significant hemolysis can cause anemia, the hemolytic effect of (2R)-2-[4-(7-bromo-2-quinolyloxy)phenoxy]propanoic acid is an undesirable property for a pharmaceutical composition.

There exists a need for formulations containing (2R)-2-[4-(7-bromo-2-quinolyloxy)phenoxy]propanoic acid being capable of reducing drug-induced hemolysis.

### SUMMARY OF THE INVENTION

Several approaches to reducing drug-induced hemolysis have been reported to be effective for certain drugs. For example, longer infusion times (i.e. slower infusion rates or decreased concentrations of active drug) for a parenteral formulation may result in decreased hemolytic activity of the active drug. However, longer infusion times may undesirably lead to decreased patient compliance. Other approaches include the addition of a tonicity agent, a surfactant, a colloid-osmotic protectant, a chelator/membrane stabilizer, a protein, a hydroxyl free radical scavenger, or a complexation agent, such as a cyclodextrin, to a formulation. However, reports describing these other approaches were primarily limited to *in vitro* results without confirmation with *in vivo* studies.

The present inventors have therefore conducted intensive studies to find an improved formulation for (2R)-2-[4-(7-bromo-2-quinolyloxy)phenoxy]propanoic acid that can reduce drug-induced hemolysis. Among the parenteral additives studied, only cyclodextrins, which form complexes with (2R)-2-[4-(7-bromo-2-quinolyloxy)phenoxy]propanoic acid, proved to be effective.

While cyclodextrins have been reported to reduce drug-induced hemolysis *in vitro,* such reduction does not necessarily predict activity in *in vivo* environments, especially during IV infusion. It is believed that cyclodextrins provide hemolytic protection through the formation of inclusion complexes with at least a portion of drug, resulting in less free drug that can interact with erythrocytes. This mechanism may apply to *in vitro* tests where there is no significant dilution of the drug solution (such as ≥ 0.9 ml of solution mixing with 0.1 ml of erythrocyte suspension), which may keep a portion of drug in complexed form. However, during IV infusion, most drugs disassociate from cyclodextrin cavities rapidly and completely upon administration to the dynamic blood stream due to extensive dilution. This makes the *in vivo* effectiveness unpredictable.

The present invention is based on the discovery that adding a cyclodextrin to a pharmaceutical formulation of (2R)-2-[4-(7-bromo-2-quinolyloxy)phenoxy]propanoic acid reduces drug-induced hemolysis of (2R)-2-[4-(7-bromo-2-quinolyloxy)phenoxy]propanoic acid in both *in vitro* and *in vivo* conditions, without affecting the pharmacological activity of the drug.

The present invention also provides methods of treatment comprising administration of the pharmaceutical formulations of the instant invention.

### DETAILED DESCRIPTION OF THE INVENTION

### Definitions and Abbreviations

As used above, and throughout the description of the invention, the following abbreviations and symbols, unless otherwise indicated, shall be understood to have the following meanings:
- α: alpha
- β: beta
- γ: gamma
- δ: delta
- IV: intravenous
- qs: quantity sufficient, i.e. a sufficient quantity to achieve a total volume indicated

As used above, and throughout the description of the invention, the following terms, unless otherwise indicated, shall be understood to have the following meanings:
The terms "active ingredient" and "active principle," as used herein, refer to (2R)-2-[4-(7-bromo-2-quinolyloxy)phenoxy]propanoic acid or pharmaceutically acceptable salts thereof.
Cyclodextrins are oligosaccharides containing a toroidal, hydrophobic central cavity and a hydrophilic outer surface. The term "cyclodextrin," as used herein, may refer to a cyclodextrin or a cyclodextrin derivative.
Captisol^{®} is a sulfobutyl ether betacyclodextrin available from CyDex, LLC.
The term "hemolysis" refers to the alteration or destruction of red blood cells in such a manner that hemoglobin is liberated into the blood stream.
The term "solid tumor cancer" is used in its medically accepted sense, and does not include cancers of the blood such as leukemias.

The invention relates to pharmaceutically acceptable aqueous formulations comprising (2R)-2-[4-(7-bromo-2-quinolyloxy)phenoxy]propanoic acid or one of its pharmaceutically acceptable salts, a physiologically acceptable cyclodextrin (including combinations of cyclodextrins), and at least one solubility-enhancing agent; the formulation generally having a pH between about 4 and about 9, preferably having a pH between about 5 and about 8.

(2R)-2-[4-(7-Bromo-2-quinolyloxy)phenoxy]propanoic acid, or one of its pharmaceutically acceptable salts, is present in the pharmaceutical formulations of the invention in a proportion of about 0.1% to about 5% (w/v), for example in a proportion of about 0.2% to about 3% (w/v). However, preferred formulations contain from about 0.5% to about 2% (w/v) of (2R)-2-[4-(7-bromo-2-quinolyloxy)phenoxy]propanoic acid or of one of its pharmaceutically acceptable salts.

Any suitable cyclodextrin can be used to carry out the present invention, including alpha-cyclodextrins, beta-cyclodextrins, gamma-cyclodextrins, and delta-cyclodextrins, and which cyclodextrins may be in the form of derivatives such as sulfoalkylether cyclodextrins (e.g., sulfobutyl ether β-cyclodextrin), hydroxyalkyl cyclodextrins, (e.g., hydroxypropyl-β-cyclodextrin, hydroxyethyl-β-cyclodextrin), alkylcyclodextrins (e.g., methyl-β-cyclodextrin, dimethyl-β-cyclodextrin, trimethyl-β-cyclodextrin, diethyl-β-cyclodextrin), or carboxyalkylcyclodextrins (e.g., carbomethyl-β-cyclodextrin), and combinations thereof. It is preferable to use sulfobutyl ether β-cyclodextrin or hydroxy-propyl β-cyclodextrin.

The above cyclodextrins make it possible in particular to reduce or prevent drug induced hemolysis of (2R)-2-[4-(7-bromo-2-quinolyloxy)phenoxy]propanoic acid or of its pharmaceutically acceptable salts by formation of an encapsulation complex of this active principle.

Generally, the cyclodextrin is incorporated in the formulations in an amount of from about 1% to about 50% (w/v), preferably from about 5% to about 20% (w/v), for example 10% (w/v). It is preferred that the cyclodextrin be present in an amount effective to substantially reduce hemolysis caused by (2R)-2-[4-(7-bromo-2-quinolyloxy)phenoxy]propanoic acid or a pharmaceutically acceptable salt thereof. By "substantially reduce," it is meant that the cyclodextrin is present in an amount effective to reduce hemolysis of the active principle by about 30% or more relative to the amount of hemolysis caused by the active principle in the absence of the cyclodextrin. In a preferred aspect of the invention, the cyclodextrin is present in an amount effective to reduce hemolysis of the active principle by about 50% or more relative to the amount of hemolysis caused by the active principle in the absence of the cyclodextrin.

Due to the limited solubility of (2R)-2-[4-(7-bromo-2-quinolyloxy)phenoxy]-propanoic acid in water, the pharmaceutical formulations contain at least one solubility-enhancing agent. A solubility-enhancing agent of the present invention is an agent or agents that enhance(s) the solubility of the active principle in the aqueous formulation. Suitable solubility-enhancing agents include pharmaceutically acceptable pH adjusting agents and/or buffering agents capable of dissolving the active principle and/or maintaining solution at a physiologically acceptable range, for example, acids/acidic agents, such as citric acid, lactic acid, boric acid, acetic acid, phosphoric acid, hydrochloric acid, sulfuric acids, and sodium phosphate monobasic; and bases/basic agents, such as sodium hydroxide, sodium citrate, sodium borate, sodium acetate, sodium sulfate, sodium phosphate dibasic, sodium phosphate tribasic, sodium carbonate, sodium bicarbonate, tris-hydroxymethylaminomethane, diethylamine, triethylamine, and ammonium hydroxide.

Preferably, the solubility-enhancing agent is a physiologically acceptable buffering agent. Generally, the buffering agent is capable both of dissolving the active principle and of maintaining the pH of the formulation between about 4 and about 9, preferably between about pH 5 and about pH 8. Preferred buffering agents of the invention include, for example, buffer systems chosen from succinic acid/alkali metal succinate, citric acid/alkali metal citrate, tartaric acid/alkali metal tartrate, lactic acid/alkali metal lactate, maleic acid/alkali metal maleate, acetic acid/alkali metal acetate, fumaric acid/alkali metal fumarate methanesulphonic acid/alkali metal methanesulphonate, alkali metal sulfates, alkali metal hydrogen sulfates, phosphate acid/monoalkali metal phosphate, alkali metal dihydrogen phosphate/dialkali metal hydrogen phosphate, trialkali metal citrate, alkali metal phosphate, akali metal carbonate/alkali metal hydrogen carbonate; the alkali metal in each of the above salts being, for example, sodium or potassium. A preferred buffering agent is monoalkali metal phosphate/dialkali metal phosphate, for example monosodium phosphate/disodium phosphate.

Preferred buffer solutions include about 0.01 to about 0.3 molar, more preferably about 0.05 to about 0.2 molar, aqueous buffer solutions of alkali metal dihydrogen phosphate/dialkali metal hydrogen phosphate, for example monosodium phosphate/disodium phosphate.

Additional solubility enhancing agents include pharmaceutically acceptable cosolvents (e.g., ethanol, propylene glycol and the like), surfactants (e.g., Polysorbate 80 and polaxamer), and polymers (e.g., polyvinylpyrrolidone).

In addition to the active principle, the cyclodextrin, the solubility-enhancing agent(s), and water, the formulations according to the invention may include tonicity modifiers, for example electrolytes such as sodium chloride, calcium chloride, non-reducing sugars, and sugar alcohols such as mannitol, sorbitol, xylitol or glycerin.

The pharmaceutical formulations according to the present invention may optionally include antioxidants (e.g., sodium bisulfite, sodium thiosulfate, and ascorbic acid).

The formulation according to the invention may also include one or more preservatives. Any suitable preservative may be used to carry out the present invention, including but not limited to benzalkonium chloride, benzyl alcohol, cresol, parabens, phenol, and thimerosal. The amount of preservative will in general be present in the amount of about 0.1 % to about 1%.

The pharmaceutical formulations and compositions of the invention can be prepared using conventional techniques known to those skilled in the art.

The pharmaceutical formulations of the present invention preferably contain a therapeutically effective amount of the active principle. The term "therapeutically effective amount," as used herein, refers to an amount of the active principle present in the pharmaceutical formulation being administered that is sufficient to elicit the desired pharmacological or therapeutic effect(s) and/or to prevent development of or alleviate to some extent one or more of the symptoms of the disease being treated. In determining the effective amount or dose, a number of factors are considered by the attending diagnostician, including, but not limited to: the species of mammal; its size, age, and general health; the specific disease involved; the degree of involvement or the severity of the disease; the response of the individual patient; the mode of administration; the bioavailability characteristics of the preparation administered; the dose regimen selected; the use of concomitant medication; and other relevant circumstances.

The pharmaceutical formulations of the present invention are generally administered to patients, which include, but are not limited to, mammals, for example, humans, by, intravenous administration (including IV bolus injection and IV infusion), intramuscular administration, and other parenteral routes. Preferably, the pharmaceutical formulations are administered by IV infusion.

Compositions can be prepared wherein the formulations of the invention are spray dried or lyophilized to form a powder for constitution. The compositions can be reconstituted with an aqueous liquid to form a parenteral formulation. Such compositions, comprising (2R)-2-[4-(7-bromo-2-quinolyloxy)phenoxy]propanoic acid or a pharmaceutically acceptable salt thereof, a physiologically acceptable cyclodextrin, and at least one solubility-enhancing agent, are a further aspect of the present invention.

Prior to administration, the pharmaceutical formulations and/or compositions can be diluted by commonly used intravenous fluids known to those of skill in the art. The pharmaceutical formulations may accordingly be prepared in a more concentrated form than that which is described above, and which may later be diluted to the desired concentration.

In another embodiment, the present invention relates to dosage forms comprising the pharmaceutical formulations described herein. Each dosage should contain the quantity of active principle calculated to produce the desired therapeutic effect. Typically, the pharmaceutical formulations will be administered in dosage units containing from about 10 mg to about 4000 mg of the active principle by weight of the composition, with a range of about 100 mg to about 2000 mg being preferred.

It will also be apparent to those skilled in the art that the pharmaceutical formulations of the present invention can be administered with other therapeutic and/or prophylactic agents and/or medicaments that are not medically incompatible therewith.

All components of the present formulations must be pharmaceutically acceptable. As used herein, a "pharmaceutically acceptable" component is one that is suitable for use with humans and/or other animals without undue adverse side effects (such as toxicity, irritation and allergic response) commensurate with a reasonable benefit/risk ratio.

The present invention further relates to the use of the pharmaceutical formulations of the invention in medicine.

(2R)-2-[4-(7-Bromo-2-quinolyloxy)phenoxy]propanoic acid is an anti-tumor agent effective against solid tumors. The present invention therefore provides therapeutic methods of treating solid tumor cancers, which comprise administering to a patient in need of such treatment a therapeutically effective amount of the formulation of the invention. A patient includes a primate, human, rodent, canine, feline, bovine, ovine, equine, swine, caprine, and the like. Solid tumor cancers include, but are not limited to, colon cancer, breast cancer, prostate cancer, ovarian cancer, melanoma, and pancreatic cancer.

A subject of the present invention is the use of a formulation of the present invention in the manufacture of medicinal products for the treatment of solid tumor cancers, such as colon cancer, breast cancer, prostate cancer, ovarian cancer, melanoma, and pancreatic cancer.

The present invention also provides methods of reducing the hemolysis activity of (2R)-2-[4-(7-bromo-2-quinolyloxy)phenoxy]propanoic acid or a pharmaceutically acceptable salt thereof, comprising adding a cyclodextrin to a composition comprising (2R)-2-[4-(7-bromo-2-quinolyloxy)phenoxy]propanoic acid or a pharmaceutically acceptable salt thereof.

The following examples will further illustrate the invention, without, however, limiting it thereto.

### In Vitro Studies

### Example 1: Assessment of in vitro hemolytic potential with dog erythrocytes.

The following study was performed to evaluate the hemolytic potential of solutions of (2R)-2-[4-(7-bromo-2-quinolyloxy)phenoxy]propanoic acid in a 0.1 M pH 8 phosphate bufferred vehicle at concentrations of 10 mg/mL and 20 mg/mL. Saline (0.9% NaCl) was used as a negative control, and saponin (3% aqueous solution), which is a known inducer of hemolysis, was used as the positive control.

Dog whole blood was mixed in a 1:1 ratio and a 9:1 ratio with solutions of the active ingredient in the vehicle and with the vehicles alone. Dog whole blood was also mixed in a 1:1 ratio and a 9:1 ratio with a 3% saponin solution as the positive control and saline (0.9% NaCl) as the negative control.

After mixing, tubes were incubated for 30 minutes at 37°C, then centrifuged (3000 t/min.) for 10 minutes. Hemoglobin concentrate was dosed in the supernatant by spectrophotometry UV/visible (Genesis technologies) for the assessment of the *in vitro* hemolytic potential. The percent of hemolysis was calculated based on the results of the saponin samples.

The results of this experiment are presented in Table 1. The 10 and 20 mg/mL solutions of the active ingredient induced hemolysis of dog erythrocytes, while the vehicle alone did not. These results indicate hemolysis induced by (2R)-2-[4-(7-bromo-2-quinolyloxy)phenoxy]propanoic acid at concentrations of 10 mg/mL and 20 mg/mL.

**Table 1: Percentage of Hemolysis***

| Blood/Solution ratio | 10 mg/mL Active Ingredient | 20 mg/mL Active Ingredient | Vehicle without Active ingredient | Saline |
|---|---|---|---|---|
| 1:1, n=5 (mean) | 24.0% | 28.0% | 2.4% | 0% |
| 9:1, n=5 (mean) | 4.5% | 6.6% | 0% | 2.4% |

| | | | | |
|---|---|---|---|---|
| **In vitro* hemolysis is expressed as the percentage of that induced by saponin. | | | | |

### Example 2: In vitro compatibility of various formulations with dog whole blood

This study was performed to evaluate the hemolytic reducing potential of various additives reported to reduce drug-induced hemolysis for certain other drugs as compared to the conventional phosphate buffer formulation described in Example 1. Specifically, the following experiment assessed the hemolytic potential of (2R)-2-[4-(7-bromo-2-quinolyloxy)phenoxy]propanoic acid in various vehicles *in vitro* at concentrations of 10 and 20 mg/mL with dog whole blood. The aqueous vehicle solutions tested were a 2% sucrose solution; a 1% human albumin solution; a 0.6% Poloxamer 188 solution; a 1% mannitol solution; a solution composed of 1% mannitol, 0.5% glycine and 0.6% poloxamer 188; a 0.5% glycine solution; and a phosphate buffer solution as the control.

Dog whole blood was mixed in a 1:1 ratio and a 9:1 ratio with solutions of the active ingredient in the various vehicles and with the vehicles alone. Whole blood was also mixed with a saponin solution in the same proportion to use as the positive control. After a 45-minute incubation at 37°C, the samples were centrifuged. The resulting supernatants were analyzed for plasma hemoglobin absorbance on a microplate reader (Dynex technologies, MRX revelation 4.06).

The percent of hemolysis was calculated based on the results of the saponin samples. An induced hemolysis was considered significant when the hemoglobin concentration was greater than 2-fold higher than the negative control value obtained by mixing whole blood with homologous plasma in a 1:1 and 9:1 ratio of whole blood to plasma (intrinsic plasma hemoglobin).

The results of this experiment are presented in Table 2. These results indicate that drug-induced hemolysis of (2R)-2-[4-(7-bromo-2-quinolyloxy)phenoxy]propanoic acid is not reduced by using additives previously described to reduce drug-induced of other drugs.

**Table 2: Mean Percentage of Hemolysis* (n=12)**

| Vehicle | Active ingredient conc. 0 mg/mL 1:1 ratio | Active ingredient conc. 10 mg/mL 1:1 ratio | Active ingredient conc. 20 mg/mL 1:1 ratio | Active ingredient conc. 0 mg/mL 9:1 ratio | Active ingredient conc. 10 mg/mL 9:1 ratio | Active ingredient conc. 20 mg/mL 9:1 ratio |
|---|---|---|---|---|---|---|
| Sucrose 2% | 4.3% | 47.4% | 77.9% | 5.2% | 7.2% | 13.0% |
| Human Albumin 1% | 2.5% | 38.4% | 77.9% | 6.5% | 5.9% | 15.0% |
| Poloxamer 188 (0.6%) | 3.2% | 45.5% | 72.4% | 7.1% | 7.9% | 15.5% |
| Mannitol 1% | 4.7% | 56.8% | 82.2% | 6.2% | 6.1% | 12.7% |
| Mannitol 1%, Glycine 0.5%, Poloxamer 188 (0.6%) | 2.0% | 35.0% | 82.1% | 3.6% | 5.6% | 13.4% |
| Glycine 0.5% | 2.1% | 34.2% | 80.2% | 3.6% | 5.1% | 15.0% |
| Phosphate buffer | 3.1% | 29.5% | 85.6% | 5.2% | 5.6% | 12.8% |
| Saponin solution 5% | 100.0% | - | - | 100.0% | - | - |
| Patient plasma | 2.7% | - | - | 5.4% | - | - |

| | | | | | | |
|---|---|---|---|---|---|---|
| **In vitro* hemolysis is expressed as the percentage of that induced by saponin. | | | | | | |

### Example 3: Assessment of in vitro hemolytic potential in dog erythrocytes for various formulations

This study was also performed to evaluate the hemolytic reducing potential of various parenteral additives reported to reduce drug-induced hemolysis for other drugs as compared to the conventional phosphate buffer formulation.

The following formulations were prepared:
A) 2 mg/mL, 5 mg/mL, and 20 mg/mL active ingredient in 0.1M pH 8 phosphate buffer;
B) 20 mg/mL active ingredient in 0.12 M sodium bicarbonate solution; and
C) 2 mg/mL, 5 mg/mL, and 20 mg/mL active ingredient, plus 10 mg/mL NaCl in 0.1M pH 8 phosphate buffer.

Saline (0.9% NaCl) was used as a negative control, and saponin (3% aqueous solution) was used as the positive control.

Dog whole blood was mixed in a 1:1 ratio and a 9:1 ratio with solutions of the active ingredient in the vehicle and with the vehicles alone. Dog whole blood was also mixed in a 1:1 ratio and a 9:1 ratio with a 3% saponin solution as the positive control and saline (0.9% NaCl) as the negative control.

After mixing, tubes were incubated for 30 minutes at 37°C, then centrifuged (3000 t/min.) for 10 minutes. Hemoglobin concentrate was dosed in the supernatant by spectrophotometry UV/visible (Genesis technologies) for the assessment of the *in vitro* hemolytic potential. The percent of hemolysis was calculated based on the results of the saponin samples.

The results of this experiment are presented in Table 3. All three formulations induced an *in vitro* hemolysis of dog erythrocytes. The index of hemolysis was expressed as a percentage of the positive control value for the saponin samples.

**Table 3: Percentage of Hemolysis***

| Formulation | Blood/Solution ratio | Vehicle without Active ingredient | 20 mg/mL Active Ingredient |
|---|---|---|---|
| A n=6 (mean) | 1:1 | 2.3% | 76.6% |
| A n=6 (mean) | 9:1 | 1.6% | 2.9% |
| B n=6 (mean) | 1:1 | 20.0% | 56.5% |
| B n=6 (mean) | 9:1 | 3.1% | 2.3% |
| C n=6 (mean) | 1:1 | 2.4% | 85.4% |
| C n=6 (mean) | 9:1 | 1.1% | 1.8% |
| Saline n=6 (mean) | 1:1 | 1.93% | - |
| Saline n=6 (mean) | 9:1 | 2.0% | - |

| | | | |
|---|---|---|---|
| **In vitro* hemolysis is expressed as the percentage of that induced by saponin. | | | |

### Example 4: Assessment of in vitro hemolytic potential in dog erythrocytes for a formulation containing a cyclodextrin.

The following study was performed to evaluate the hemolytic reducing potential of a formulation containing 10 mg/mL active ingredient as a solution in a 0.05M pH 7 phosphate buffer formulation with 10% (w/v) hydroxypropyl beta cyclodextrin. Saline (0.9% NaCl) was used as the negative control, and saponin (3% aqueous solution) was used as the positive control.

Dog whole blood was mixed in a 1:1 ratio and a 9:1 ratio with solutions of the active ingredient in the vehicle and with the vehicles alone. Dog whole blood was also mixed in a 1:1 ratio and a 9:1 ratio with a 3% saponin solution as the positive control and saline (0.9% NaCl) as the negative control.

After mixing, tubes were incubated for 30 minutes at 37°C, then centrifuged (3000 t/min.) for 10 minutes. Hemoglobin concentrate was dosed in the supernatant by spectrophotometry UV/visible (Genesis technologies) for the assessment of the *in vitro* hemolytic potential. The percent of hemolysis was calculated based on the results of the saponin samples.

The results of this experiment are presented in Table 4. The 10 mg/mL cyclodextrin formulation of the invention *did not* induce an *in vitro* hemolysis of dog erthrocytes.

**Table 4: Percentage of Hemolysis***

| Blood/Solution ratio | 10 mg/mL Active Ingredient | Vehicle without Active ingredient | Saline |
|---|---|---|---|
| 1:1, n=6 (mean) | 1.8% | 0.2% | 1.8% |
| 9:1, n=6 (mean) | 0.5% | 0.8% | 1.2% |

| | | | |
|---|---|---|---|
| **In vitro* hemolysis is expressed as the percentage of that induced by saponin. | | | |

### Example 5: In vitro study of cyclodextrin formulation with human blood and plasma

The following experiment assessed the potential of a cyclodextrin formulation of the invention to produce *in vitro* hemolysis in human blood.

Active ingredient solutions at concentrations of 2 mg/mL, 5 mg/mL, and 10 mg/mL in a vehicle of 10% Captisol^{®} (w/v) in 0.05 M phosphate buffer were prepared as test solutions. For each solution, a blood to test solution and blood to vehicle ratio of 1:1 and 9:1 were tested. The final concentrations of active ingredient in the vehicles were 0.2, 0.5, 0.1, 2.5, and 5 mg/mL. Whole blood was mixed with 5% saponin as the positive control. All tubes were incubated at 37°C for 45 minutes, then centrifuged for 5 minutes at approximately 1500 g (15-20°C). The resulting supernatants were analyzed for plasma hemoglobin absorbance on a microplate reader (Dynex technologies, MRX revelation 4.06).

The percent of hemolysis was calculated based on the results of the saponin samples. Hemolysis was considered significant when the test solution value was 2-fold higher than the negative control value obtained by mixing whole blood with homologous plasma in a 1:1 and a 9:1 control.

Results: The Captisol^{®} vehicle alone and the Captisol^{®}-containing formulations of the invention, having final concentrations of 0.2, 0.5, 1, 2.5, and 5 mg/mL, *did not* induce hemolysis of human whole blood.

### In Vivo Studies

### Example 6: In vivo study in dogs with phosphate buffer formulation, 30-minute infusion

Solutions of the active ingredient in 0.1M phosphate buffer at concentrations 10 mg/mL and 20 mg/mL were administered to 1 male and 1 female beagle dog each, by a single intravenous infusion over 30 minutes. Dosages were 25 and 50 mg/kg. Sampling for plasma level determination was taken for 1 day at the following intervals: end of infusion (0), 30 minutes, 1 hour, 3 hours, 6 hours, and 24 hours after the end of the infusion.

Results: A visual hemolysis was observed on plasma samples at time 0, 30 minutes post infusions (all animals), 1 hour (male treated at 25 mg/kg and both animals treated at 50 mg/kg), 3 hours (both animals at 50 mg/kg) and 6 hours (female at 50 mg/kg).

### Example 7: In vivo studies with phosphate buffer formulation or a cyclodextrin formulation of the invention

Solutions of the active ingredient in 0.1M phosphate buffer at concentrations 0, 2.5, 5 and 10 mg/mL were administered to 10 male and 10 female rats each, by a single intravenous infusion over 1 hour. Dosages were 0 (control), 100, 200, and 400 mg/kg.

Results: On the day of the infusion, discolored urine (red/brown) was observed in 6 out of 10 males and 8 out of 10 females at the 400 mg/kg dose, which was most likely due to the hemolytic potential of the active ingredient.

In a study conducted in rats at dose of 100, 200, 300 and 350 mg/kg using a formulation with Captisol^{®}, discolored (red) urine was noted at the 350 mg/kg dose in only 3 out of 12 females on Day 1 and 1 out of 12 females on Day 2. This dose was obtained using the highest concentration (10 mg/mL) and a very high volume of administration in rats (35 mL/kg).

No hemolysis was observed in a study conducted in dogs at dose of 10, 50 and 75 mg/kg (maximal concentration of 7.5 mg/mL with a volume of administration of 10 mL/kg) using a formulation with Captisol^{®}.

### Example 8: In vivo study in dogs with a cyclodextrin formulation of the invention

A solution of the active ingredient (10 mg/mL) in an aqueous solution of 0.05 M pH 7 phosphate buffer with 10% hydroxy-propyl β-cyclodextrin was administered to a female beagle dog by a single intravenous infusion over 1-hour. The dosage was 50 mg/kg. Sampling was taken for 1 day at the following intervals: end of infusion (0), 30 minutes, 1 hour, 3 hours, 6 hours and 24 hours after the end of the infusion.

Results: No ex-vivo hemolysis was noted in the blood samplings.

### Example 9: The following are examples of pharmaceutical formulations of the present invention:

| Material | Amount |
|---|---|
| Active ingredient | 8.00 mg |
| Sulfobutyl ether betacyclodextrin | 100 mg |
| Sodium Phosphate Monobasic Dihydrate | 2.92 mg |
| Sodium Phosphate Dibasic Dodecahydrate | 10.1 mg |
| Water | qs to 1 ml |

| Material | Amount |
|---|---|
| Active ingredient | 10.0 mg |
| Sulfobutyl ether betacyclodextrin | 100 mg |
| Sodium Phosphate Monobasic Monohydrate | 2.5 mg |
| Sodium Phosphate Dibasic, Anhydrous | 3.9 mg |
| Water | qs to 1 ml |

## Claims

1. A pharmaceutically acceptable aqueous formulation comprising (2R)-2-[4-(7-bromo-2-quinolyloxy)phenoxy]propanoic acid or a pharmaceutically acceptable salt thereof, a physiologically acceptable cyclodextrin, and at least one solubility-enhancing agent.

2. The formulation according to claim 1 wherein the (2R)-2-[4-(7-bromo-2-quinolyloxy)phenoxy]propanoic acid or a pharmaceutically acceptable salt thereof is present in an amount of from about 0.1% to about 5% (w/v).

3. The formulation according to claim 1, wherein the cyclodextrin is selected from the group consisting of alpha-cyclodextrins, beta-cyclodextrins, gamma-cyclodextrins, delta-cyclodextrins, and derivatives thereof.

4. The formulation according to claim 3, wherein the cyclodextrin is selected from the group consisting of sulfoalkylether cyclodextrins, hydroxyalkyl cyclodextrins, alkylcyclodextrins, and carboxyalkylcyclodextrins.

5. The formulation according to claim 4, where the cyclodextrin is selected from the group consisting of sulfobutyl ether β-cyclodextrin and hydroxy-propyl β-cyclodextrin.

6. The formulation according to claim 5, wherein the cyclodextrin is sulfobutyl ether β-cyclodextrin.

7. The formulation according to claim 6, wherein the cyclodextrin is Captisol^{®}

8. The formulation according to claim 1, wherein the cyclodextrin is present in an amount of from about 1% to about 50% (w/v).

9. The formulation according to claim 1, wherein the solubility-enhancing agent is a physiologically acceptable buffering agent.

10. The formulation according to claim 9, wherein the buffering agent concentration is about 0.01M to about 0.25M.

11. The formulation according to claim 9, wherein the buffering agent is a monoalkali metal phosphate/dialkali metal phosphate buffer.

12. The formulation according to claim 1 having a pH between about 5.0 and about 8.0.

13. The formulation according to claim 1 further comprising a tonicity modifier.

14. The formulation according to claim 1 further comprising one or more physiologically acceptable preservatives.

15. The formulation according to claim 1 further comprising a pharmaceutically acceptable excipient.

16. A composition comprising (2R)-2-[4-(7-bromo-2-quinolyloxy)phenoxy]propanoic acid or a pharmaceutically acceptable salt thereof, a physiologically acceptable cyclodextrin, and at least one solubility-enhancing agent.

17. The composition according to claim 16, wherein the cyclodextrin is sulfobutyl ether β-cyclodextrin.

18. A method for treating a solid tumor cancer, which comprises administering to a patient in need of such treatment a therapeutically effective amount of a pharmaceutical formulation according to claim 1.

19. The method according to claim 18, wherein the solid tumor cancer is selected from the group consisting of colon cancer, breast cancer, prostate cancer, ovarian cancer, melanoma, and pancreatic cancer.

20. Use of the pharmaceutical formulation according to claim 1 in the manufacture of a medicinal product for the treatment of a solid tumor cancer.

21. A method of reducing hemolysis activity of (2R)-2-[4-(7-bromo-2-quinolyloxy)phenoxy]propanoic acid or a pharmaceutically acceptable salt thereof, comprising adding a physiologically acceptable cyclodextrin to a composition comprising (2R)-2-[4-(7-bromo-2-quinolyloxy)phenoxy]propanoic acid or a pharmaceutically acceptable salt thereof.

22. The method according to claim 21 wherein said cyclodextrin is selected from the group consisting of sulfobutyl ether β-cyclodextrin and hydroxy-propyl β-cyclodextrin.
